# EUROPEAN PATENT SPECIFICATION

(11) **EP 3 182 991 B1**
(45) Date of publication and mention of the grant of the patent: **08.07.2020**
(21) Application number: 15770811.6
(22) Date of filing: 20.08.2015
(51) Int. Cl.: A61K 38/18, C07K 14/475, A61P 35/00

(54) **VEGF FOR ENHANCING THE PERMEABILITY OF BLOOD-BRAIN BARRIER**
VEGF ZUR VERBESSERUNG DER DURCHLÄSSIGKEIT DER BLUT-HIRN-SCHRANKE
VEGF POUR AMÉLIORER LA PERMÉABILITÉ DE LA BARRIÈRE HÉMATO-ENCÉPHALIQUE

(30) Priority: 20.08.2014 US 201462039899 P
(43) Date of publication of application: 28.06.2017
(73) Proprietor: Academia Sinica, Taipei, 115 (TW)
(72) Inventor: HSIEH, Patrick C.H., Taipei 115 (TW); LUNDY, David, Washington Tyne and Wear NE37 1PZ (GB)
(74) Representative: Cabinet Becker et Associés
(86) International application number: PCT/EP2015/069184
(87) International publication number: WO 2016/026942

(56) References cited:
- WO-A1-2009/108632
- WO-A1-2012/104822
- WO-A1-2012/168721
- WO-A2-2004/096254
- SHIZE JIANG ET AL: "Vascular Endothelial Growth Factors Enhance the Permeability of the Mouse Blood-brain Barrier", PLOS ONE, vol. 9, no. 2, 14 February 2014 (2014-02-14), page e86407, XP055225371, DOI: 10.1371/journal.pone.0086407
- ZHENG GANG ZHANG ET AL: "VEGF enhances angiogenesis and promotes blood-brain barrier leakage in the ischemic brain", JOURNAL OF CLINICAL INVESTIGATION, vol. 106, no. 7, 1 October 2000 (2000-10-01), pages 829-838, XP055225450, US ISSN: 0021-9738, DOI: 10.1172/JCI9369

### SEQUENCE LISTING

<110> Academia Sinica et al.
<120> METHODS FOR ENHANCING PERMEABILITY TO BLOOD-BRAIN BARRIER AND USES THEREOF
<130> B2079PC00
<160> 1
<170> PatentIn version 3.5
<210> 1
   <211> 165
   <212> PRT
   <213> homo sapiens
<400> 1
   Ala Pro Met Ala Glu Gly Gly Gly Gln Asn His His Glu Val Val Lys
   1               5                   10                  15
   Phe Met Asp Val Tyr Gln Arg Ser Tyr Cys His Pro Ile Glu Thr Leu
               20                  25                  30
   Val Asp Ile Phe Gln Glu Tyr Pro Asp Glu Ile Glu Tyr Ile Phe Lys
           35                  40                  45
   Pro Ser Cys Val Pro Leu Met Arg Cys Gly Gly Cys Cys Asn Asp Glu
       50                  55                  60
   Gly Leu Glu Cys Val Pro Thr Glu Glu Ser Asn Ile Thr Met Gln Ile
   65                  70                  75                  80
   Met Arg Ile Lys Pro His Gln Gly Gln His Ile Gly Glu Met Ser Phe
                   85                  90                  95
   Leu Gln His Asn Lys Cys Glu Cys Arg Pro Lys Lys Asp Arg Ala Arg
               100                 105                 110
   Gln Glu Asn Pro Cys Gly Pro Cys Ser Glu Arg Arg Lys His Leu Phe
           115                 120                 125
   Val Gln Asp Pro Gln Thr Cys Lys Cys Ser Cys Lys Asn Thr Asp Ser
       130                 135                 140
   Arg Cys Lys Ala Arg Gln Leu Glu Leu Asn Glu Arg Thr Cys Arg Cys
   145                 150                 155                 160
   Asp Lys Pro Arg Arg
                   165

## Description

### BACKGROUND OF INVENTION

The blood-brain barrier (BBB) comprises a network of capillary endothelial cells linked by tight junctions, restricting the free exchange of small molecules, proteins and cells between systemic circulation and the CNS. The BBB protects the brain from pathogens, toxins and other insults but represents a challenging obstacle in the treatment of many neurological disorders. Pathologies of the brain such as tumors, infection, infarction/haemorrhage, physical trauma and degenerative diseases (e.g., Parkinson's disease) are common and serious disorders where adequate drug delivery and accurate diagnostic imaging are critical.

Central nervous system (CNS) diseases often result in serious morbidity, death or impairment of mobility, due to limited surgical or medical therapy that is currently available. Although an expanding number of potential therapeutic compounds exist for treating these disorders, the lack of suitable approaches to deliver these agents to the CNS, particularly, the brain, limits their uses. Currently available delivery techniques rely on systemic, intrathecal or intra-cranial drug administration; however, all of them have limitations. For example, the inability of many compounds to cross from the circulatory system to the CNS restrict systemic delivery. Even if systemic delivered agents enter the CNS, the amount of such agents is often too low to elicit desirable therapeutic responses.

Accordingly, there exists a need to develop new approaches to facilitate delivery of therapeutic and diagnostic agents across the BBB for treating or diagnosing brain diseases.

### SUMMARY OF INVENTION

The present disclosure is based, at least in part, on the discoveries that VEGF facilitates the delivery of agents, including small molecules, macromolecules, nanoparticles, and stem cells, across the blood-brain barrier to the brain when a low dose of VEGF was given within a suitable time window (*e.g*., 45 minutes) prior to the administration of the agents.

Accordingly, one aspect of the present disclosure features a method for treating brain tumor, comprising: (i) administering systemically to a subject having a brain tumor a vascular endothelial growth factor (VEGF); and (ii) administering systemically an effective amount of an anti-cancer agent to the subject within 5 hours after the administration of VEGF.

In some examples, the amount of VEGF can be 5 to 200 ng/kg, for example, 25 ng/kg. Alternatively or in addition, the anti-cancer agent can be administered 15-180 minutes after the administration of VEGF, for example, 45 minutes or 3 hours after the administration of VEGF.

The anti-cancer agent may be an alkylating agent (e.g., cisplatin, carboplatin, oxaliplatin, mechlorethamine, cyclophosphamide, melphalan, chlorambucil, ifosfamide, busulfan, N-nitroso-N-methylurea (MNU), carmustine, lomustine, semustine, fotemustine, streptozotocin, dacarbazine, mitozolomide, temozolomide, thiotepa, mytomycin, and diaziquone); a topoisomerase inhibitor (e.g., camptothecin, irinotecan, topotecan, etoposide, doxorubicin, teniposide, novobiocin, merbarone, and aclarubicin); an anti-metabolite (e.g., fluoropymidine, deoxynucleoside analogue, thiopurine, methotrexate, and pemetrexed); a cytotoxicity antibiotic (actinomycin, bleomycin, plicamycin, mitomycin, doxorubicin, daunorubicin, epirubicin, idarubicin, piraubicin, alcarubicin, and mitoxantrone), or a biologic (e.g., a therapeutic antibody such as Bevacizumab, Cetuximab, Pemtumomab, oregovomab, minretumomab, Etaracizumab, Volociximab, Cetuximab, panitumumab, nimotuzumab, Trastuzumab, pertuzumab, AVE1642, IMC-A12, MK-0646, R1507, CP 751871, Mapatumumab, KB004 or IIIA4).

In another aspect, the present disclosure provides a method for facilitating delivery of an agent (e.g., a therapeutic agent or a diagnostic agent such as a contrast agent) across the blood-brain barrier of a subject, comprising administering systemically to a subject in need thereof an effective amount of vascular endothelial growth factor (VEGF) within 5 hours prior to administration of the agent, wherein the effective amount of VEGF is 5 to 200 ng/kg (e.g., 25 ng/kg), and wherein the agent is a therapeutic agent or a diagnostic agent. In some examples, the VEGF is administered 15 minutes to 3 hours prior to the administration of the agent, for example, 45 minutes or 3 hours prior to the administration of the agent.

In any of the methods described herein, the subject can be a human patient having, suspected of having, or at risk for a brain disease. Examples of brain diseases include, but are not limited to, ischemic stroke, a neurodegenerative disease, and a neuropsychiatric disorder.

When a diagnostic agent is used, the method may further comprise detecting the presence or level of the diagnostic agent in a brain area of the subject. The diagnostic agent can be detected by computed tomography (CT) or magnetic resonance imaging (MRI).

Further, the present disclosure provides a kit comprising: (i) a first container containing a first formulation that comprises a vascular endothelial growth factor (VEGF), and (ii) a second container containing a second formulation that comprises a therapeutic agent (e.g., an anti-cancer agent as those described herein) or a diagnostic agent, e.g., as those described herein. Such a kit can be used for treating or diagnosing a brain disorder such as a brain tumor, wherein both the first formulation and the second formulation may be for systematical administration to a subject in need of the treatment and wherein the first formulation may be administered within 5 hours before administration of the second formulation.

Also within the scope of the present disclosure is a pharmaceutical composition for co-use with a therapeutic agent or a diagnostic agent for treating or diagnosing a brain disease, the pharmaceutical composition comprising VEGF, wherein the pharmaceutical composition is administered to a subject in need thereof within 5 hours prior to administration of the therapeutic agent or the diagnostic agent, and wherein the amount of VEGF administered to the subject is 5 to 200 ng/kg.

The details of one or more embodiments of the invention are set forth in the description below. Other features or advantages of the present invention will be apparent from the following drawings and detailed description of several embodiments, and also from the appended claims.

### BRIEF DESCRIPTION OF DRAWINGS

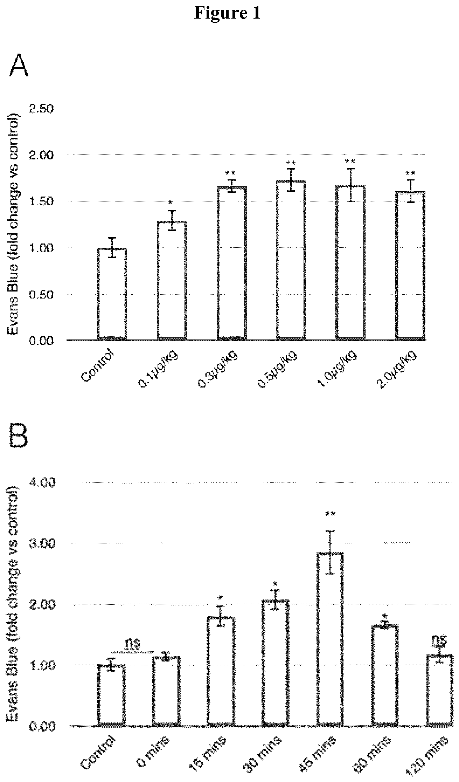

**Figure 1** includes charts showing that systemic injection of VEGF increased the permeability of the blood brain barrier in mice. A. Effect of VEGF doses on BBB permeability, measured by Evans Blue content of brain tissue. * = P < 0.05 versus control; and ** = P < 0.05 versus VEGF 0.1 µg/kg group. B. Time course of BBB permeability after systemic infection of 0.3µg/kg VEGF, measured by Evans Blue. ns = not significant; * = P < 0.05 versus control group; and ** = P < 0.05 versus VEGF 30 min group. All bars show mean ± SD. n = 6 for all groups.

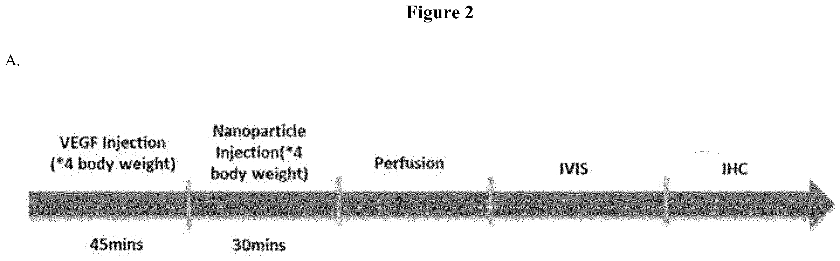

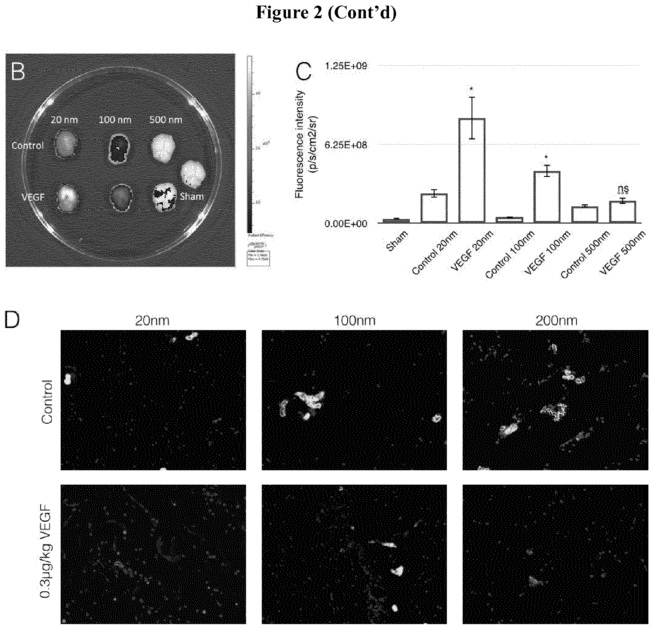

**Figure 2** includes diagrams showing that VEGF pre-treatment increased penetration of PEGylated fluorescent nanoparticles through the BBB in mice. A. A flow chart showing experimental design. B. IVIS images of nanoparticle biodistribution in the brain with and without VEGF pretreatment. C. A chart showing the quantification of fluorescence intensity by IVIS analysis. D. A photo showing immunofluorescence staining of tissue sections that indicate the biodistribution of different sized nanoparticles in the brain following VEGF pretreatment. Mice were pre-treated with 0.3 µg/Kg VEGF injection, held for 45 min, then PEGylated nanoparticles (20, 100 or 500 nm) were injected, fluorescent intensity was then measured by IVIS analysis. ns represents not significant, * represents P < 0.05 versus control. All bars represent mean + SD, n=6 for all groups.

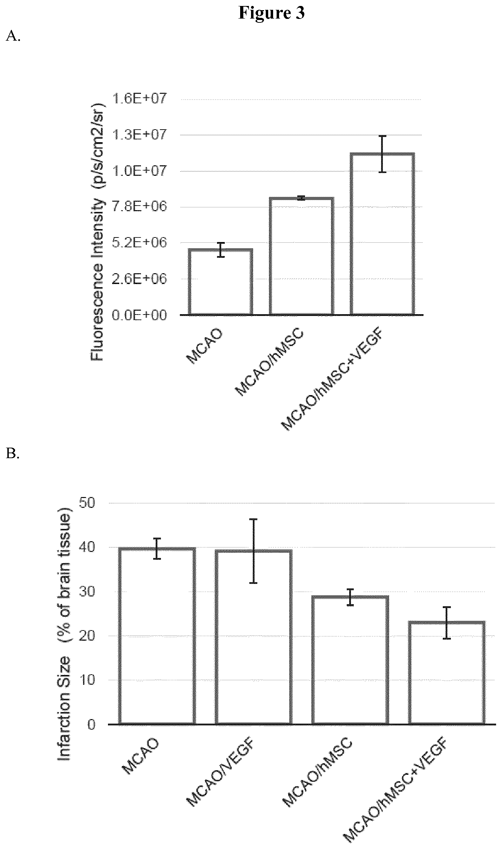

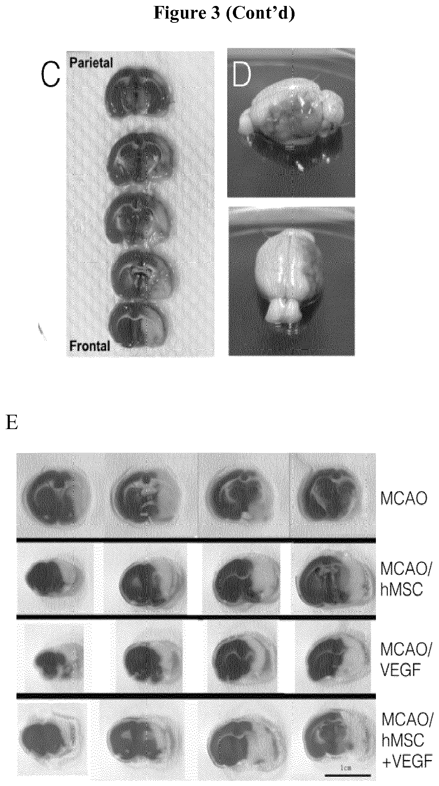

**Figure 3** includes diagrams showing that VEGF pre-treatment enhanced post-stroke hMSC-based cell therapy. A. A chart showing the quantitative fluorescent intensity measured in emitted MCAO rats after treatment with Ds-Red expressing hMSCs with or without 0.3 µg/Kg VEGF pre-treatment. B. A chart showing the infarction size in MCAO rat after treatment of Ds-Red expressing hMSCs with or without 0.3 µg/Kg VEGF pre-treatment. C. A photo showing a MCAO model with 2,3,5-triphenyltetrazolium chloride (TTC) staining. The pale region is the infarcted area. D. A photo showing Evans Blue staining indication retention of dye in the infarcted area. E. A photo showing MCAO rat brain slices with decreased infarction size in both hMSC and hMSC/VEGF treatment groups after 3 days.

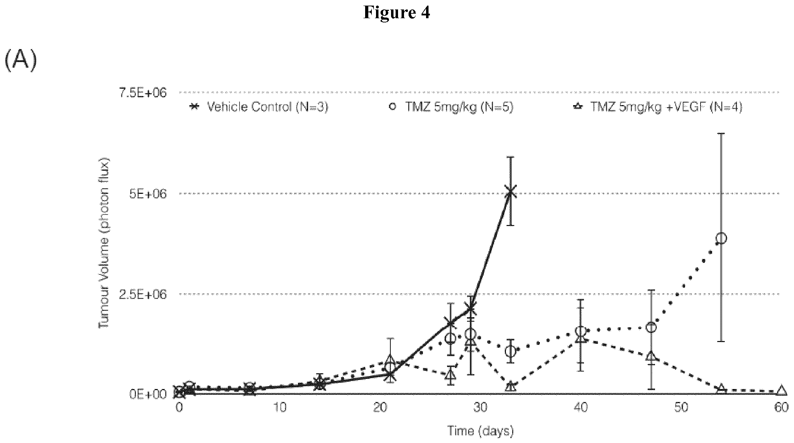

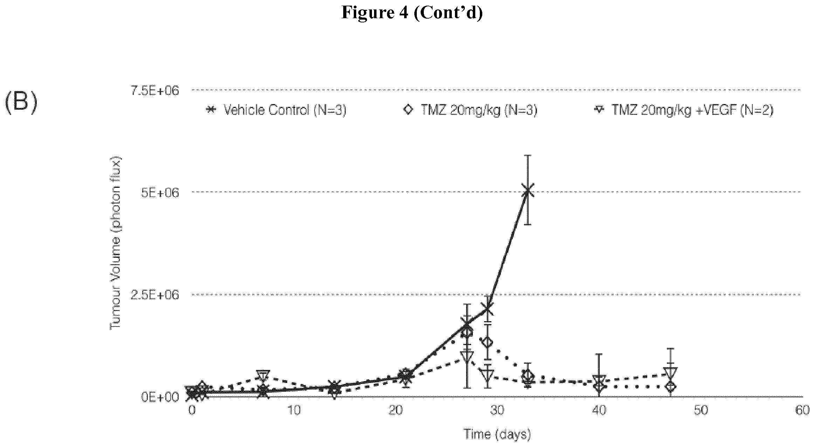

**Figure 4** includes charts showing that VEGF pre-treatment followed by temozolomide (TMZ) injection effectively delayed GBM tumor growth in a GBM Mouse Model. A. Tumor volume after treatment with 5 mg/Kg TMZ with or without 0.3 µg/Kg VEGF pre-treatment. B. Tumor volume after treatment with 20 mg/Kg TMZ with or without 0.3 µg/Kg VEGF pre-treatment.

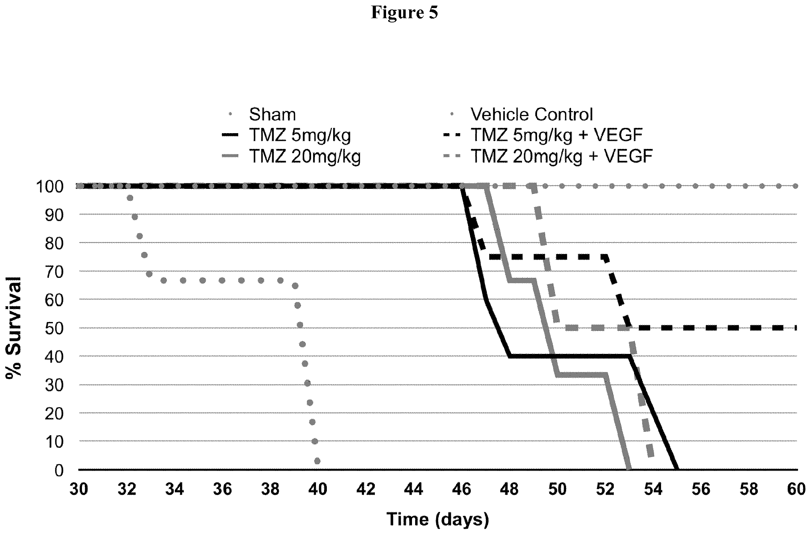

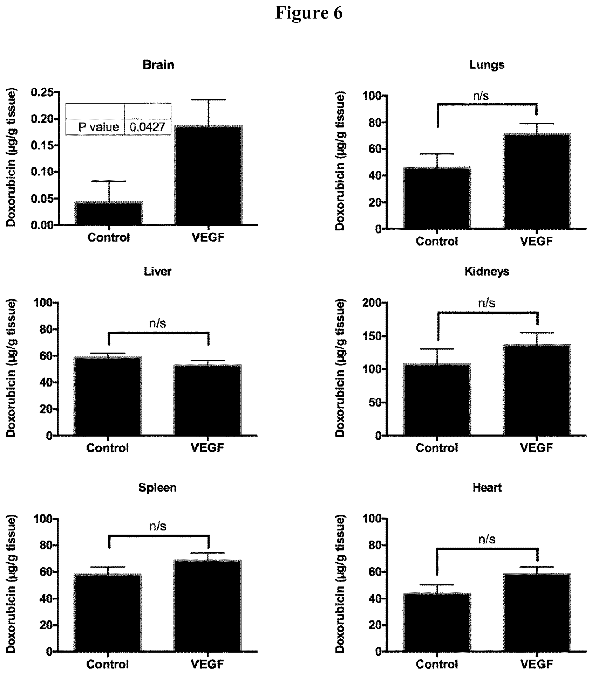

**Figure 5** is a graph showing that VEGF pre-treatment improves GBM mice survival. **Figure 6** includes graphs showing doxorubicin isolated from six vital organs, brain, lung, liver, kidney, spleen, and heart, in VEGF165A (0.3 µg/kg) or control (normal saline) pre-treated mice. A significant increase in doxorubicin measured in the brain was detected following VEGF treatment with no changes in the other vital organs. n=3 per group.

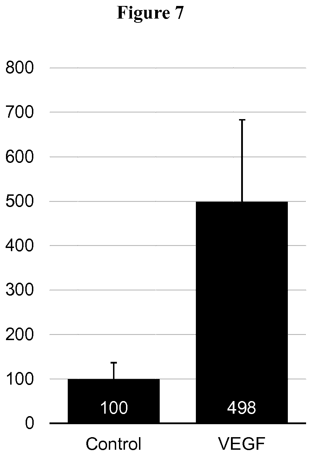

**Figure 7** is a graph showing a quantitative analysis of the intensity of fluorescent signals in the brain of mice injected intravenously with either 0.3 µg/kg VEGF165A or normal saline control 45 minutes prior to injection with an anti-nrCAM antibody. The result shows an approximately 5-fold increase in signal intensity following VEGF treatment.

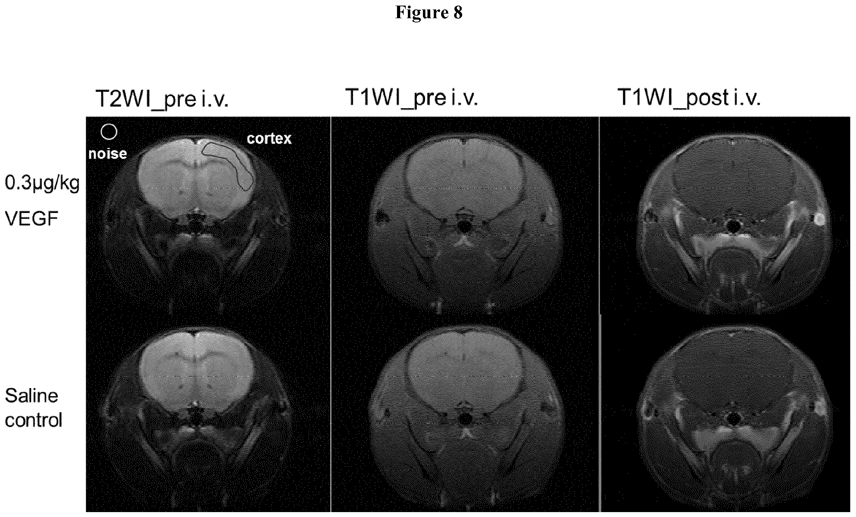

**Figure 8** includes images showing that VEGF pre-treatment followed by injection of gadolinium contrast agent increased the detectable levels of contrast agent in the mouse brain as compared to saline control. The result shows an approximate 15.5% increase in the amount of gadolinium detected in the brain of mice pre-treated with VEGF as compared to mice pre-treated with saline control.

### DETAILED DESCRIPTION OF INVENTION

A number of approaches have been tried to overcome the challenges associated with drug delivery across the BBB, including disruption of the BBB, permeating the BBB, bypassing the BBB, or a combination thereof. Osmotic treatments can disrupt the barrier, and many attempts have been made to utilize endogenous carrier proteins for drug uptake and delivery. The BBB may be avoided entirely by direct injection of drugs into cerebrospinal fluid or directly into the brain. However, these methods present their own challenges such as ion imbalances, leaking neurotransmitters and chemokine release into circulation. Obermeier et al., Nat Med 19(12): 1584-1596; 2013.

Vascular endothelial growth factor (VEGF) is a signal protein produced by cells that stimulates vasculogenesis and angiogenesis. It is a growth factor that belongs to the platelet-derived growth factor sub-family. The normal function of VEGF is to create new blood vessels during embryonic development, new blood vessels after injury, muscle following exercise, and new vessels (collateral circulation) to bypass blocked vessels. The mammalian VEGF family comprises five members: VEGF-A, placenta growth factor (PGF), VEGF-B, VEGF-C and VEGF-D. There are multiple isoforms in some of the VEGF families (e.g., VEGF A) due to alternative splicing.

Because angiogenesis is closely related to tumor growth and metastasis and VEGF promotes angiogenesis, VEGF was suggested to play a role in tumorigenesis. For example, it was reported that VEGF-mediated signaling not only lead to angiogenesis and vascular permeability, but also contribute to key aspects of tumorigenesis, including cancer stem cells and tumor initiation. Goel et al., Nature Reviews Cancer 13, 871-882 (2013).

The present disclosure is based, at least in part, on the discovery that VEGF temporarily increased BBB permeability by approximately three-fold, peaking 45 minutes after administration and returning to normal permeability after 2 hours. Further, pre-treatment of VEGF-A165 in two animal models enhanced Temozolomide (TMZ) delivery to aggressive brain tumors and human mesenchymal stem cell (hMSC)-based therapy to reduce infarction damage after stroke. The efficacy of treating glioblastoma by TMZ (*e.g.,* reduced tumor volume and enhanced survival rate) was improved when the animal was pre-treated with VEGF at a low dose, which were unexpected given the role of VEGF in tumorigenesis as known in the art. Moreover, the present data indicated that, surprisingly, VEGF not only facilitated delivery of small molecular drugs (e.g., TMZ and doxorubicin) across the BBB, but also enhanced delivery of large agents, including antibodies, nanoparticles, and stem cells, across the BBB. The results provided herein indicate that systemic administration of VEGF at a low dose within a suitable time window prior to the administration of a therapeutic or diagnostic agent could temporarily increase the permeability of the BBB to these agents, thereby enhancing the delivery of the agents the brain.

Accordingly, provided herein are methods for enhancing treatment or diagnosis efficacy of brain diseases by the co-use of VEGF and a therapeutic or diagnostic agent, wherein the VEGF may be systemically delivered at a low lose within a suitable time window prior to the administration of the therapeutic/diagnostic agent; and kits for performing such methods.

### DEFINITIONS

For convenience, certain terms employed in the context of the present disclosure are collected here. Unless defined otherwise, all technical and scientific terms used herein have the same meaning as commonly understood by one of the ordinary skill in the art to which this invention belongs.

The singular forms "a", "and", and "the" are used herein to include plural referents unless the context clearly dictates otherwise.

The term "treatment" as used herein are intended to mean obtaining a desired pharmacological and/or physiologic effect, e.g., delaying or inhibiting cancer growth or ameliorating ischemic injury to an organ (e.g., brain). The effect may be prophylactic in terms of completely or partially preventing a disease or symptom thereof and/or therapeutic in terms of a partial or complete cure for a disease and/or adverse effect attributable to the disease. "Treatment" as used herein includes preventative (e.g., prophylactic), curative or palliative treatment of a disease in a mammal, particularly human; and includes: (1) preventative (e.g., prophylactic), curative or palliative treatment of a disease or condition (e.g., a cancer or heart failure) from occurring in an individual who may be pre-disposed to the disease but has not yet been diagnosed as having it; (2) inhibiting a disease (e.g., by arresting its development); or (3) relieving a disease (e.g., reducing symptoms associated with the disease).

The term "administered", "administering" or "administration" are used interchangeably herein to refer a mode of delivery, including, without limitation, intraveneously, intramuscularly, intraperitoneally, intraarterially, intracranially, or subcutaneously administering an agent (e.g., a compound or a composition) of the present invention. In one embodiment of the present disclosure, the growth factor (e.g., VEGF), the therapeutic agent or the contrast agent for imaging is administered to the subject by direct intraveneously or intracranially injection. Systemic administration is a route of administration of an agent into the circulatory system so that the entire body is affected. Administration can take place via enteral administration (absorption of the drug through the gastrointestinal tract) or parenteral administration (injection, infusion, or implantation).

The term "an effective amount" as used herein refers to an amount effective, at dosages, and for periods of time necessary, to achieve the desired result with respect to the treatment of a disease. For example, in the treatment of a cancer, an agent (i.e., a compound or a composition) which decrease, prevents, delays or suppresses or arrests any symptoms of the cancer would be effective. An effective amount of an agent is not required to cure a disease or condition but will provide a treatment for a disease or condition such that the onset of the disease or condition is delayed, hindered or prevented, or the disease or condition symptoms are ameliorated. The effective amount may be divided into one, two or more doses in a suitable form to be administered at one, two or more times throughout a designated time period.

The term "subject" or "patient" refers to an animal including the human species that is treatable with the method of the present invention. The term "subject" or "patient" intended to refer to both the male and female gender unless one gender is specifically indicated. Accordingly, the term "subject" or "patient" comprises any mammal which may benefit from the treatment method of the present disclosure.

The terms "tumor" and "cancer " are used interchangeably herein, and is intended to mean any cellular malignancy whose unique trait is the loss of normal controls that results in unregulated growth, lack of differentiation and/or ability to invade local tissues and metastasize. Human brain tumors include, but are not limited to, gliomas, metastases, meningiomas, pituitary adenomas, and acoustic neuromas. Examples of gliomas include astrocytoma, pilocytic astrocytoma, low-grade astrocytoma, anaplastic astrocytoma, glioblastoma multiforme, brain stem glioma, ependymoma, subependymoma, ganglioneuroma, mixed glioma, oligodendroglioma, and optic nerve glioma. Examples of non-glial tumors include acoustic neuroma, chordoma, CNS lymphoma, craniopharyngioma, hemangioblastoma, medulloblastoma, meningioma, pineal tumors, pituitary tumors, primitive neuroectodermal tumors (PNET), rhabdoid tumors, and schwannoma. Tumors that affect the cranial nerves include gliomas of the optic nerve, neurofibromas of 8th cranial nerve, neurofibromas of 5th cranial nerve. Benign tumors include arachnoid, dermoid, epidermoid, colloid, and neuroepithelial cysts and any other slow growing tumors. While primary brain tumors, like those described above, originate in the brain itself, metastatic brain tumors (secondary brain tumors that begin as cancer in another part of the body) are the most common brain tumors. Cerebral metastases can spread from primary cancers including, but not limited to, cancers originating in the lung, skin (melanoma), kidney, colon and breast.

The term "stroke" as used herein is intended to mean any event that blocks or reduces blood supply to all or part of the brain. Stroke may be caused by thrombosis, embolism or hemorrhage, and may be referred to as ischemic stroke (including thrombotic stroke and embolic stroke and resulting from thrombosis, embolism, systemic hypo-perfusion, and the like) or hemorrhagic stroke (resulting from intracerebral hemorrhage, subarachnoid hemorrhage, subdural hemorrhage, epidural hemorrhage, and the like). As used herein, stroke excludes heat-stroke and transient ischemic attacks (TIA). Heat-stroke results from an elevated temperature in the body and its clinical manifestations in the brain are different from those of stroke as defined herein (i.e., interruption of blood supply associated with reduced oxygen in the brain). TIA are sometimes referred to as "mini-strokes," however they can be distinguished from stroke as defined herein due to their ability to resolve completely within 24 hours of occurrence. Stroke is diagnosed through neurological examination, blood tests, and/or medical imaging techniques such as Computed Tomography (CT) scans (e.g., without contrast agents), Magnetic Resonance Imaging (MRI) scans, Doppler ultrasound, and arteriography.

The term "neuropsychiatric disorder" is intended to mean a neurological disturbance that is typically labeled according to which of the four mental faculties are affected. For example, one group includes disorders of thinking and cognition, such as schizophrenia and delirium; a second group includes disorders of mood, such as affective disorders and anxiety; a third group includes disorders of social behavior, such as character defects and personality disorders; and a fourth group includes disorders of learning, memory, and intelligence, such as mental retardation and dementia. Accordingly, neuropsychiatric disorders of the present disclosure encompass schizophrenia, delirium, Alzheimer's disease (AD), depression, mania, attention deficit disorders (ADD), attention deficit hyperactivity disorder (ADHD), drug addiction, mild cognitive impairment, dementia, agitation, apathy, anxiety, psychoses, post-traumatic stress disorders, irritability, and bipolar disorder.

The term "neurodegenerative disease" as used herein refers to a condition characterized by the death of neurons in different regions of the nervous system and the consequent functional impairment of the affected subjects. Neurodegenerative disease of the present disclosure encompasses Alzhemer's disease (AD), argyrophilic grain disease, amyotrophic lateral sclerosis (ALS), ALS-parkinsonism dementia complex of Guam, vascular dementia, frontotemporal dementia, semantic dementia, dementia with Lewy bodies, Huntington's disease, inclusion body myopathy, inclusion body myositis, or Parkinson's disease (PD).

### Use of VEGF to Facilitate Delivery of Therapeutic/Diagnostic Agents Across the BBB

One aspect of the present disclosure features methods of treating or diagnosing brain diseases that involve the co-use of a VEGF and a therapeutic or diagnostic agent, wherein the VEGF is systemically administered at a low dose within a suitable time window prior to the administration of the therapeutic or diagnostic agent. Besides VEGF, other growth factors such as IGF-I and IGF-II, may also be used in the methods described herein.

### (i) VEGF

VEGF of any of the five families noted herein can be used for the method disclosed herein. The VEGF can be from a suitable origin, e.g., human, monkey, mouse, rat, pig, dog, and cat. In some embodiments, the VEGF molecule used in the methods described herein is a VEGF-A molecule, such as the VEGF-A₁₆₅ isoform. The amino acid sequence of the human VEGF-A₁₆₅ is:

In some instances, the VEGF molecule used in the methods described herein is a wild-type VEGF. In other instances, it can be a modified variant, which preserves the same or similar bioactivity as the wild-type counterpart.

Such a modified variant may share a sequence identity of at least 85% *(e.g.,* 90%, 95%, 97%, 99%, or above) relative to the wild-type counterpart. The "percent identity" of two amino acid sequences is determined using the algorithm of Karlin and Altschul Proc. Natl. Acad. Sci. USA 87:2264-68, 1990, modified as in Karlin and Altschul Proc. Natl. Acad. Sci. USA 90:5873-77, 1993. Such an algorithm is incorporated into the NBLAST and XBLAST programs (version 2.0) of Altschul, et al. J. Mol. Biol. 215:403-10, 1990. BLAST protein searches can be performed with the XBLAST program, score=50, wordlength=3 to obtain amino acid sequences homologous to the protein molecules of interest. Where gaps exist between two sequences, Gapped BLAST can be utilized as described in Altschul et al., Nucleic Acids Res. 25(17):3389-3402, 1997. When utilizing BLAST and Gapped BLAST programs, the default parameters of the respective programs (e.g., XBLAST and NBLAST) can be used.

In some embodiments, the modified variant consists of one or more conservative amino acid residue substitutions as compared with the wild-type counterpart. The skilled artisan will realize that conservative amino acid substitutions may be made in a VEGF molecule to provide functionally equivalent variants, i.e., the variants retain the functional capabilities of the particular VEGF. As used herein, a "conservative amino acid substitution" refers to an amino acid substitution that does not alter the relative charge or size characteristics of the protein in which the amino acid substitution is made. Variants can be prepared according to methods for altering polypeptide sequence known to one of ordinary skill in the art such as are found in references which compile such methods, e.g. Molecular Cloning: A Laboratory Manual, J. Sambrook, et al., eds., Second Edition, Cold Spring Harbor Laboratory Press, Cold Spring Harbor, New York, 1989, or Current Protocols in Molecular Biology, F.M. Ausubel, et al., eds., John Wiley & Sons, Inc., New York. Conservative substitutions of amino acids include substitutions made amongst amino acids within the following groups: (a) M, I, L, V; (b) F, Y, W; (c) K, R, H; (d) A, G; (e) S, T; (f) Q, N; and (g) E, D.

Conservative amino-acid substitutions in the amino acid sequence of a VEGF to produce functionally equivalent variants typically are made by alteration of a nucleic acid encoding the mutant. Such substitutions can be made by a variety of methods known to one of ordinary skill in the art. For example, amino acid substitutions may be made by PCR-directed mutation, site-directed mutagenesis according to the method of Kunkel (Kunkel, PNAS 82: 488-492, 1985), or by chemical synthesis of a nucleic acid molecule encoding a VEGF variant.

Any of the VEGF molecules for use in the methods described herein may be prepared by conventional methods. For example, the molecule can be isolated from a suitable natural source following the routine protein purification procedures. Alternatively, it can be produced in a suitable host cell via the conventional recombinant technology.

### (ii) Pharmaceutical Compositions

Any of the active agents for use in the methods described herein (e.g., the VEGF, the therapeutic agent, and the diagnostic agent) can be mixed with a pharmaceutically acceptable carrier (excipient), including buffer, to form a pharmaceutical composition for use in inhibiting sclerostin expression, enhancing osteoblast differentiation, and/or promoting bone fracture healing. "Acceptable" means that the carrier must be compatible with the active ingredient of the composition (and preferably, capable of stabilizing the active ingredient) and not deleterious to the subject to be treated. Pharmaceutically acceptable excipients (carriers) including buffers, which are well known in the art. See, e.g., Remington: The Science and Practice of Pharmacy 20th Ed. (2000) Lippincott Williams and Wilkins, Ed. K. E. Hoover.

The pharmaceutical compositions to be used in the present methods can comprise pharmaceutically acceptable carriers, excipients, or stabilizers in the form of lyophilized formulations or aqueous solutions. (Remington: The Science and Practice of Pharmacy 20th Ed. (2000) Lippincott Williams and Wilkins, Ed. K. E. Hoover). Acceptable carriers, excipients, or stabilizers are nontoxic to recipients at the dosages and concentrations used, and may comprise buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrans; chelating agents such as EDTA; sugars such as sucrose, mannitol, trehalose or sorbitol; salt-forming counter-ions such as sodium; metal complexes (e.g. Zn-protein complexes); and/or non-ionic surfactants such as TWEEN™, PLURONICS™ or polyethylene glycol (PEG). Pharmaceutically acceptable excipients are further described herein.

In one example, one or more of the active agents may be formulated into liquid pharmaceutical compositions, which are sterile solutions, or suspensions that can be administered by, for example, intravenous, intramuscular, subcutaneous, or intraperitoneal injection. Suitable diluents or solvent for manufacturing sterile injectable solution or suspension include, but are not limited to, 1,3-butanediol, mannitol, water, Ringer's solution, and isotonic sodium chloride solution. Fatty acids, such as oleic acid and its glyceride derivatives are also useful for preparing injectables, as are natural pharmaceutically-acceptable oils, such as olive oil or castor oil. These oil solutions or suspensions may also contain alcohol diluent or carboxymethyl cellulose or similar dispersing agents. Other commonly used surfactants such as Tweens or Spans or other similar emulsifying agents or bioavailability enhancers that are commonly used in manufacturing pharmaceutically acceptable dosage forms can also be used for the purpose of formulation.

In some examples, the pharmaceutical composition described herein comprises liposomes containing one of the active agent (e.g., VEGF), which can be prepared by methods known in the art, such as described in Epstein, et al., Proc. Natl. Acad. Sci. USA 82:3688 (1985); Hwang, et al., Proc. Natl. Acad. Sci. USA 77:4030 (1980); and U.S. Pat. Nos. 4,485,045 and 4,544,545. Liposomes with enhanced circulation time are disclosed in U.S. Pat. No. 5,013,556. Particularly useful liposomes can be generated by the reverse phase evaporation method with a lipid composition comprising phosphatidylcholine, cholesterol and PEG-derivatized phosphatidylethanolamine (PEG-PE). Liposomes are extruded through filters of defined pore size to yield liposomes with the desired diameter.

The active agents (e.g., an VEGF molecule) may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are known in the art, see, e.g., Remington, The Science and Practice of Pharmacy 20th Ed. Mack Publishing (2000).

The pharmaceutical compositions to be used for *in vivo* administration must be sterile. This is readily accomplished by, for example, filtration through sterile filtration membranes. Therapeutic compositions are generally placed into a container having a sterile access port, for example, an intravenous solution bag or vial having a stopper pierceable by a hypodermic injection needle.

The pharmaceutical compositions described herein can be in unit dosage forms such as tablets, pills, capsules, powders, granules, solutions or suspensions, or suppositories, for oral, parenteral or rectal administration, or administration by inhalation or insufflation.

For preparing solid compositions such as tablets, the principal active ingredient can be mixed with a pharmaceutical carrier, e.g. conventional tableting ingredients such as corn starch, lactose, sucrose, sorbitol, talc, stearic acid, magnesium stearate, dicalcium phosphate or gums, and other pharmaceutical diluents, e.g. water, to form a solid preformulation composition containing a homogeneous mixture of a compound of the present invention, or a non-toxic pharmaceutically acceptable salt thereof. When referring to these preformulation compositions as homogeneous, it is meant that the active ingredient is dispersed evenly throughout the composition so that the composition may be readily subdivided into equally effective unit dosage forms such as tablets, pills and capsules. This solid preformulation composition is then subdivided into unit dosage forms of the type described above containing from 0.1 to about 500 mg of the active ingredient of the present invention. The tablets or pills of the novel composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer that serves to resist disintegration in the stomach and permits the inner component to pass intact into the duodenum or to be delayed in release. A variety of materials can be used for such enteric layers or coatings, such materials including a number of polymeric acids and mixtures of polymeric acids with such materials as shellac, cetyl alcohol and cellulose acetate.

Suitable surface-active agents include, in particular, non-ionic agents, such as polyoxyethylenesorbitans (e.g. Tween™ 20, 40, 60, 80 or 85) and other sorbitans (e.g. Span™ 20, 40, 60, 80 or 85). Compositions with a surface-active agent will conveniently comprise between 0.05 and 5% surface-active agent, and can be between 0.1 and 2.5%. It will be appreciated that other ingredients may be added, for example mannitol or other pharmaceutically acceptable vehicles, if necessary.

Suitable emulsions may be prepared using commercially available fat emulsions, such as Intralipid™, Liposyn™, Infonutrol™, Lipofundin™ and Lipiphysan™. The active ingredient may be either dissolved in a pre-mixed emulsion composition or alternatively it may be dissolved in an oil (e.g. soybean oil, safflower oil, cottonseed oil, sesame oil, corn oil or almond oil) and an emulsion formed upon mixing with a phospholipid (e.g. egg phospholipids, soybean phospholipids or soybean lecithin) and water. It will be appreciated that other ingredients may be added, for example glycerol or glucose, to adjust the tonicity of the emulsion. Suitable emulsions will typically contain up to 20% oil, for example, between 5 and 20%. The fat emulsion can comprise fat droplets between 0.1 and 1.0 .im, particularly 0.1 and 0.5 .im, and have a pH in the range of 5.5 to 8.0.

The emulsion compositions can be those prepared by mixing a VEGF or a therapeutic/diagnostic agent with Intralipid™ or the components thereof (soybean oil, egg phospholipids, glycerol and water).

Pharmaceutical compositions for inhalation or insufflation include solutions and suspensions in pharmaceutically acceptable, aqueous or organic solvents, or mixtures thereof, and powders. The liquid or solid compositions may contain suitable pharmaceutically acceptable excipients as set out above. In some embodiments, the compositions are administered by the oral or nasal respiratory route for local or systemic effect.

Compositions in preferably sterile pharmaceutically acceptable solvents may be nebulised by use of gases. Nebulised solutions may be breathed directly from the nebulising device or the nebulising device may be attached to a face mask, tent or intermittent positive pressure breathing machine. Solution, suspension or powder compositions may be administered, preferably orally or nasally, from devices which deliver the formulation in an appropriate manner.

### (iii) Therapeutic Applications

VEGF (as well as other growth factors) can be co-used with a therapeutic agent or a diagnostic agent for treating or diagnosing a brain disease, including brain tumor, brain stroke, a neuropsychiatric disorder, or a neurodegenerative disease, e.g., those described herein. The method described herein can also be applied for brain imaging.

To perform the methods described herein, a pharmaceutical composition comprising a VEGF (e.g., human VEGF-A165) and a pharmaceutical composition comprising a suitable therapeutic or diagnostic agent can be administered to any subject in need of the treatment (e.g., as those described herein) sequentially. The VEGF may be administered within a suitable time window prior to the administration of the therapeutic or diagnostic agent. For example, the VEGF is administered less than 5 hours prior to the administration of the therapeutic or diagnostic agent. In some embodiments, the VEGF is administered 15-180 minutes (*e.g.,* 15-120, 15-90, 15-60, 30-120, 30-90, or 30-60 minutes) prior to the administration of the therapeutic or diagnostic agent. In some embodiments, the growth factor is administered 15, 20, 25, 30, 35, 40, 45 or 50 min prior to the administration of the therapeutic or diagnostic agent. In one example, the VEGF is administered 45 minutes the administration of the therapeutic or diagnostic agent. In another example, the administration of the VEGF is 3 hours prior to the administration of the therapeutic or diagnostic agent.

Alternatively, the growth factor and the therapeutic/diagnostic agent may be administered concomitantly.

The growth factor, as well as the therapeutic/diagnostic agent, may be administered to a mammal, preferably human, by any route that may effectively transports the growth factor and/or the therapeutic agent to the appropriate or desired site of action, such as oral, nasal, pulmonary, transdermal, such as passive or iontophoretic delivery, or parenteral, e.g., rectal, depot, subcutaneous, intravenous, intramuscular, intranasal, intra-peritoneal, intra-arterial, intra-cranial, intra-cerebella, subcutaneous, ophthalmic solution or an ointment. Further, the administration of the growth factor of this invention with the therapeutic agent may be concurrent or sequential.

In some embodiments, the growth factor such as VEGF can be administered via a conventional systemic route, for example, intravenous injection. Injectable compositions may contain various carriers such as vegetable oils, dimethylactamide, dimethyformamide, ethyl lactate, ethyl carbonate, isopropyl myristate, ethanol, and polyols (glycerol, propylene glycol, liquid polyethylene glycol, and the like). For intravenous injection, water soluble agents such as VEGF can be administered by the drip method, whereby a pharmaceutical formulation containing the agent and a physiologically acceptable excipients is infused. Physiologically acceptable excipients may include, for example, 5% dextrose, 0.9% saline, Ringer's solution or other suitable excipients. Intramuscular preparations, e.g., a sterile formulation of a suitable soluble salt form of the agent, can be dissolved and administered in a pharmaceutical excipient such as Water-for-Injection, 0.9% saline, or 5% glucose solution.

The growth factor such as VEGF may be administered to a subject at a low dose. In some examples, the VEGF is administered to a subject (e.g., a human subject) in the amount of 5 to 200 ng/kg. The selected dose of the growth factor (e.g., VEGF) should be high enough to enhance the permeability of BBB, but insufficient to disrupt the integral structure of BBB that inevitably leads to subsequent damage to the brain (e.g., edema). Accordingly, the growth factor such as VEGF is preferably to be administered to the subject (e.g., a human subject) in the amount of about 10 to 100 ng/kg, such as about 10, 15, 20, 25, 30, 35, 40, 45, 50, 55, 60, 65, 70, 75, 80, 85, 90, 95 or 100 ng/kg. Still more preferably, the growth factor is administered to the subject in the amount of about 15 to 50 ng/kg, such as about 15, 20, 25, 30, 35, 40, 45, or 50 ng/kg. Most preferably, the growth factor is administered to the subject in the amount of about 25 ng/kg.

It will be appreciated that the dosage of the growth factor and/or the therapeutic agent of the present disclosure will vary from patient to patient not only for the particular growth factor or therapeutic agent selected, the route of administration, and the ability of the growth factor or the therapeutic agent to elicit a desired response in the patient, but also factors such as disease state or severity of the condition to be alleviated, age, sex, weight of the patient, the state of being of the patient, and the severity of the pathological condition being treated, concurrent medication or special diets then being followed by the patient, and other factors which those skilled in the art will recognize, with the appropriate dosage ultimately being at the discretion of the attendant physician. Dosage regimens may be adjusted to provide the desired response. Preferably, the growth factor of the present invention is administered at an amount and for a time such that permeability to BBB is increased, then at least one dosages of the therapeutic agent are administered subsequently to the subject to achieve an improved therapeutic response.

In some embodiments, the methods described herein can be applied for treating a brain tumor such as glioblastoma (e.g., glioblastoma multiform). An anti-cancer drug such as those described herein may be co-used with a VEGF (as well as another growth factor as described herein) following the disclosures provided herein. A low dose VEGF was found to increase the BBB permeability to not only small molecule drugs but also protein drugs/nanoparticles/stem cells. Accordingly, both small-molecule anti-cancer drugs and biologics can be co-used with VEGF as described herein to enhance the treatment efficacy of the brain tumor.

In some embodiments, the methods described herein can be applied for treating a brain disorder, including, but not limited to, brain stroke, a neuropsychiatric disorder, or a neurodegenerative disease. Examples of such brain diseases are provided herein. In some examples, stem cells such as MSCs can be co-used with VEGF (as well as other growth factors as described herein) for treating brain stroke or a neurodegenerative disease following the disclosures provided herein. In other instances, an anti-coagulant (e.g., those described herein) may be co-used with VEGF for treating brain stroke. Further, an anti-psychotic or anti-dementia agent, including any of those described herein, may be co-used with VEGF for treating a psychotic disorder or dementia. Examples of these target diseases are also provided in the present disclosure.

In other embodiments, the methods described herein can be applied for brain imaging by co-use a VEGF (or other growth factors) with an imaging agent, such as a contrast agent. The contrast agent may be any agent that can be detected using computed tomography (CT) such as positron emission tomography (PET) or single photon emission computed tomography (SPECT); or magnetic resonance imaging (MRI).

### Kits for Use in Treating or Diagnosing Brain Disorders

The present disclosure also provides kits for use in the methods described herein for treating or diagnosing a brain disease. Such kits can include at least two containers, one containing a first formulation that comprises a VEGF and the other containing a second formulation that comprises a therapeutic agent as those described herein (e.g., an anti-cancer agent) or a diagnostic agent as also described herein (e.g., an imaging agent).

In some embodiments, the kit can comprise instructions for use in accordance with any of the methods described herein. The included instructions can comprise a description of administration of the VEGF and/or the therapeutic agent/diagnostic agent to treat or diagnose a target brain disease as described herein. The kit may further comprise a description of selecting an individual suitable for the treatment based on identifying whether that individual has the target disease. In still other embodiments, the instructions may comprise a description of administering the VEGF or the therapeutic/diagnostic agent to an individual at risk of the target disease.

The instructions relating to the use of a VEGF and/or the therapeutic/diagnostic agent generally include information as to dosage, dosing schedule, and route of administration for the intended treatment or diagnosis. The containers may be unit doses, bulk packages (e.g., multidose packages) or sub-unit doses. Instructions supplied in the kits described herein are typically written instructions on a label or package insert (e.g., a paper sheet included in the kit), but machine-readable instructions (e.g., instructions carried on a magnetic or optical storage disk) are also acceptable.

The label or package insert indicates that the composition is used for treating/diagnosing, delaying the onset and/or alleviating a brain disease or disorder such as those described herein. Instructions may be provided for practicing any of the methods described herein.

The kits of this invention are in suitable packaging. Suitable packaging includes, but is not limited to, vials, bottles, jars, flexible packaging (*e.g*., sealed Mylar or plastic bags), and the like. Also contemplated are packages for use in combination with a specific device, such as an inhaler, nasal administration device (*e.g.,* an atomizer) or an infusion device such as a minipump. A kit may have a sterile access port (for example the container may be an intravenous solution bag or a vial having a stopper pierceable by a hypodermic injection needle). The container may also have a sterile access port (for example the container may be an intravenous solution bag or a vial having a stopper pierceable by a hypodermic injection needle).

Kits may optionally provide additional components such as buffers and interpretive information. Normally, the kit comprises a container and a label or package insert(s) on or associated with the container. In some embodiments, the invention provides articles of manufacture comprising contents of the kits described above.

### General techniques

The practice of the present invention will employ, unless otherwise indicated, conventional techniques of molecular biology (including recombinant techniques), microbiology, cell biology, biochemistry and immunology, which are within the skill of the art. Molecular Cloning: A Laboratory Manual, second edition (Sambrook, et al., 1989) Cold Spring Harbor Press; Oligonucleotide Synthesis (M. J. Gait, ed., 1984); Methods in Molecular Biology, Humana Press; Cell Biology: A Laboratory Notebook (J. E. Cellis, ed., 1998) Academic Press; Animal Cell Culture (R. I. Freshney, ed., 1987); Introduction to Cell and Tissue Culture (J. P. Mather and P. E. Roberts, 1998) Plenum Press; Cell and Tissue Culture: Laboratory Procedures (A. Doyle, J. B. Griffiths, and D. G. Newell, eds., 1993-8) J. Wiley and Sons; Methods in Enzymology (Academic Press, Inc.); Handbook of Experimental Immunology (D. M. Weir and C. C. Blackwell, eds.); Gene Transfer Vectors for Mammalian Cells (J. M. Miller and M. P. Calos, eds., 1987); Current Protocols in Molecular Biology (F. M. Ausubel, et al., eds., 1987); PCR: The Polymerase Chain Reaction, (Mullis, et al., eds., 1994); Current Protocols in Immunology (J. E. Coligan et al., eds., 1991); Short Protocols in Molecular Biology (Wiley and Sons, 1999); Immunobiology (C. A. Janeway and P. Travers, 1997); Antibodies (P. Finch, 1997); Antibodies: a practical approach (D. Catty., ed., IRL Press, 1988-1989); Monoclonal antibodies: a practical approach (P. Shepherd and C. Dean, eds., Oxford University Press, 2000); Using antibodies: a laboratory manual (E. Harlow and D. Lane (Cold Spring Harbor Laboratory Press, 1999); The Antibodies (M. Zanetti and J. D. Capra, eds., Harwood Academic Publishers, 1995).

### Example 1: VEGF Enhanced the Permeability of Blood-Brain Barrier in a Mouse Model

### Materials and Methods

### (i) Determination of BBB Permeability by Evans Blue (EB) Extravasation

BBB permeability was quantitatively estimated by Evans Blue (Fluka). Evans Blue (2% or 4% v/v in saline, 4ml/kg) was injected intravenously. For extravasation analysis, animals were perfused with 50ml 0.9% saline (with 10 i.u./ml heparin) to remove intravascular dye until colourless fluid was obtained from the arteries, kidney and liver. Animals were then sacrificed and the brain was weighed, homogenised in N,N-dimethylformamide (Sigma-Aldrich) (1ml/150mg tissue weight), incubated for 18 hours at 55 °C, and centrifuged (14000rpm/20min). Dye supernatant was analysed by spectrophotometry at 620nm. For MCAO model rats, the brain was divided into infarcted and non-infarcted sides and supernatants from both sides were measured.

### (ii) Determination of VEGF action on BBB Permeability

Mice were divided into six groups according to VEGF dosage; Control (saline only), 0.1µg/kg, 0.3µg/kg, 05µg/kg, 1.0µg/kg and 2.0µg/kg of VEGF. The BBB permeability was determined by EB assay 1 hour after VEGF injection, as described above. The lowest dose of VEGF attaining maximum BBB permeability was selected for further experimentation. The optimum timing of VEGF and drug delivery was established by examining EB extravasation at different time points (0, 15, 30, 45, 60, 120 mins) after injection of the optimal VEGF dose.

### (iii) Nanoparticle Preparation and Injection

COOH-modified nanoparticles (20 to 500nm) were covalently modified with methoxy (MeO)-PEG-amine (NH₂) according to previously published protocols (Nance et al., Sci Transl Med 4(149): 149ra119; 2012). PEGylated fluorescent nanoparticles of 20nm, 100nm and 500nm were used to investigate the biodistribution and extravasation after intravenous injection and perfusion into FVB mice. These nanoparticles were quantified by IVIS and immunohistochemistry.

### (iv) IVIS Imaging

All IVIS images were taken using a Xenogen IVIS Spectrum device (PerkinElmer, Waltham, MA).

### (v) Statistical Analyses

GraphPad Prism 5 software was used for data analysis. The results are presented as mean with standard error of mean (SEM) or standard deviation (SD), as indicated in figure legends. One-way ANOVA was used for multiple comparisons. While comparing two groups, a one-tailed Student's t-test was used to determine statistical significance. Results were considered significant at P < 0.05.

### Results

### (i) Pre-treatment of VEGF at low doses within a time window increased BBB permeability

In this example, the time window and dosage of VEGF pre-treatment to increase BBB permeability was investigated. Mice were treated with saline (control) or VEGF165A at various doses (0.1µg/kg, 0.3 µg/Kg, 0.5 µg/Kg, 1.0 µg/kg, and 2.0 µg/kg) first, and then administered with Evan Blue (EB) at different time points (0, 15, 30, 45, 60, and 120 mins) after the VEGF treatment. BBB permeability was assessed by measuring the level of EB in the mouse brain tissue according to procedures described in "Materials and Methods" section. Results are illustrated in Figure 1.

As depicted in Figure 1, Panel A, VEGF increased BBB permeability of EB at the tested doses. The time course of BBB permeability induced by 0.3 µg/kg VEGF was further investigated, and the results are shown in Figure 1, panel B. Significant BBB permeability increase was observed when EB was injected 15 min after the injection of VEGF, and the increased permeability persisted for at least 60 min, with the maximum permeability occurred at about 45 min.

Thus, the results obtained from this study indicate that pre-treatment of VEGF at a low dose can enhance BBB permeability of agents, suggesting that low dose VEGF can be used to facilitate drug delivery across the BBB.

### (ii) VEGF pre-treatment increased the penetration of PEGvlated fluorescent nanoparticles through the BBB in mice

The effect of VEGF pre-treatment at low doses for enhancing the penetration of PEGylated fluorescent nanoparticle across the BBB in mice was investigated. Mice were first injected intravenously with a low dose of VEGF (0.3 µg/Kg). 45 minutes after the VEGF injection, the mice were injected with PEGylated nanoparticles having various sizes (20, 100 or 200 nm in diameter). The mice were subjected to perfusion following routine procedures 30 minutes after injection of the nanoparticles and the brain tissues were analyzed by IVIS imaging and immunohistochemistry. Figure 2, panel A. The amount of nanoparticles accumulated in the brain tissue (i.e., across the BBB) was determined by measuring the intensity of the fluorescent signals. Results are illustrated in Figure 2, panels B-D.

The results obtained from this study showed that pre-treatment of VEGF at a low dose facilitated the nanoparticles to cross BBB and small particles (e.g., 20nm particles) showed a higher penetration rate relative to large particles (e.g., 500nm particles).

### (iii) VEGF pre-treatment increased the penetration of an antibody through the BBB in mice

8 week old, male, FVB mice were injected intravenously with either 0.3µg/kg VEGF165A or a normal saline control 45 minutes prior to injection with an anti-nrCAM antibody, which was detected using a labelled secondary antibody to stain histological sections. The antibody was allowed to circulate for 1hr before the brain was removed and prepared for histological sectioning. As a positive control, the labeled anti-nrCAM antibody was injected directly into the brain tissue. A secondary antibody was used as a negative control. As an experimental control, normal saline was injected followed by the labeled anti-nrCAM antibody.

The mice were sacrificed and brain tissues were prepared for immunohistochemistry analysis, using the secondary antibody, which was conjugated to Alexa-488. VEGF-treated animals showed stronger signal in the brain tissue, indicating that more of the injected anti-nrCAM antibody had penetrated into the brain. Figure 7. Intensity of the fluorescent signal was quantified in ImageJ (n=3 samples per group) and background was corrected against the negative control. The result shows an approximate 5-fold increase in signal intensity following VEGF treatment.

This study indicated that low dose pre-treatment of VEGF facilitated the delivery of large molecules, such as antibodies, across the BBB.

### Example 2: VEGF Pre-Treatment Enhanced Post-Stroke hMSC-Based Cell Therapy

Brain stroke, also known as cerebrovascular accident (CVA), is the rapid loss of brain function due to disturbance in the blood supply to the brain. There are two types of stroke: hemorrhagic stroke and ischemic stroke. The hemorrhagic stroke is resulted from the disruption of intracranial arteries and thereby causing acute intracranial haematoma. The ischemic stroke, also known as cerebral infarction, is brain cell death in an area of the brain where the blood flow is blocked, such as by thrombus or distal embolism. Currently, the two standard treatments for ischemic stroke are injection of thrombolytic agents and endovascular procedures, both of which aim to re-establish local blood flow and decrease the hypoxic damage to brain tissue as quickly as possible.

Mesenchymal stem cells (MSCs) are a heterogeneous population of multipotent stromal cells, capable of differentiating into osteoblasts, chondrocytes, and adipocytes. Previous studies indicate that MSCs can activate endogenous restorative response after brain injury. Horita et al., Journal of Neuroscience Research 84(7): 1495-1504; 2006; and Li et al., Neurosci Lett 456(3): 120-123; 2009. Thus, in this example, the inventors explored the possibility of delivering (1) human mesenchymal stem cells (hMSCs), or (2) a contrast agent, across BBB with the aid of low dose VEGF, to treat (1) brain stroke or (2) to provide improved brain image for computed tomography (CT) or MRI.

A rat model of Middle Cerebral Artery Occlusion (MCAO) was used to investigate the effect of VEGF pre-treatment at a low dose on penetration of hMSCs across the BBB. The procedure published by Boyko and colleagues (Boyko, et al., Journal of Neuroscience Methods 193(2): 246-253; 2010) was used to create a middle cerebral artery occlusion (MCAO) model in rats. In brief, the left side carotid bifurcation was exposed with a self-retractor. After carefully separating the internal cerebral artery (ICA) from peripheral soft tissue, a small opening was created at the proximal ICA. Silicon-coated 4-0 monofilament nylon was inserted from the opening and penetrated forward to the beginning of middle cerebral artery (MCA). An O2C oximeter was used to transcranially monitor the ipsilateral MCA blood flow. Once blood flow decreased successfully, the nylon suture was fixed in situ, mimicking ischaemic stroke. After 90 minutes of ischaemic injury, the suture was removed, allowing reperfusion. A stable and reproducible cerebral infarction could be created with this procedure.

For hMSC cell therapy, 2x10⁶ hMSCs in 800µl of α-MEM culture medium were administered 90 minutes after ischaemic brain injury. For all VEGF treatments, 03µg/kg of VEGF was administrated intravenously immediately after ischaemic injury. MCAO model rats were divided into four groups: MCAO only, MCAO with hMSCs, MCAO with VEGF treatment only, and MCAO with VEGF pre-treatment followed by hMSC therapy. In the MCAO group with VEGF pre-treatment, hMSCs were administered 35 minutes after VEGF pre-treatment.

Discosoma sp. Red (Ds-Red)- expressing human mesenchymal stem cells (hMSCs) were used for MCAO cell therapy. Cells were routinely cultured and maintained in DMEM with 10% FBS, at 37°C with 5% CO₂.

To evaluate the infarction size, rats were sacrificed three days after ischaemic-reperfusion injury and treatment. The brains were extracted and sliced into 2mm thick coronal sections and stained with 2% 2,3,5-Triphenyl Tetrazolium Chloride (TTC) for 20 minutes. Brain slices were then scanned and the infarction size was calculated by measuring the pale area with ImageJ software. Figure 3, panels C-E.

The MCAO were treated with Ds-Red expressing hMSCs 35 minutes after VEGF pre-treatment at a low dose. Quantitated fluorescent intensity emitted from the Ds-Red confirmed that more hMSCs were delivered to the brain after the stroke in mice pre-treated with the low dose VEGF as compared to control mice. Figure 3, panel A. VEGF pre-treatment also reduced the brain infarction. Figure 3, panel B.

Results of this example affirmed that low dose VEGF can increase the permeability of BBB in a subject for about 1 hr, with the maximum BBB permeability occurred at about 45 min after VEGF pre-treatment. These results indicate that low dose VEGF can facilitate the delivery of therapeutic or diagnostic agents across the BBB to the CNS.

### Example 3: VEGF Pre-Treatment Delayed Brain Tumor Growth in Mice Treated with Anti-Cancer Agents and Improved GBM Mice Survival

Glioblastoma multiform (GBM), also known as grade IV glioma, is the most common and most aggressive type of malignant tumour of brain tissue. The current standard for treatment is a combination of surgical resection, radiotherapy and chemotherapy. GBM is highly invasive and infiltrates healthy tissue, and most patients are diagnosed when the tumor is too large and disseminated for surgical removal. Therefore, even when undergoing modern treatments, the median survival rate for GBM patients is 12-15 months after commencing treatment, one of the lowest 5-year survival rates of all human cancers. Since surgical resection is rarely successful, drug therapies are the most promising area for improvement. Suitable anti-cancer drugs already exist; however, the BBB interrupts drug delivery to brain tumors. Patel et al., J Neurooncol. 61(3): 203-207; 2003.

This study investigated the effect of low dose VEGF pre-treatment on delivery of anti-cancer drugs, TMZ and doxorubicin, across the BBB in a mouse GBM model.

The human luciferase-expressing glioblastoma cell line U-87MG was used for all GBM experiments. Cells were routinely cultured in EMEM with 10% FBS, at 37°C with 5% CO₂.

BALB/c nude mice were orthotropically xenografted with U87 glioma cells (2x10⁵) by stereotactic (2 mm right and 1 mm posterior to the bregma, 3.5 mm deep from the dura) injection into the brain. Mouse tumour progression was measured by IVIS at different time points (0, 1, 3, 7...every 7 days until 60 days).

The DNA methylating drug temozolomide (TMZ) was used as therapy for GBM model mice. 6-8 week old BALB/c-nu/nu mice were divided into four groups; Sham, Vehicle control (saline), TMZ (5mg/kg in saline), and TMZ with VEGF pre-treatment (0.3µg/kg). Tumor growth was monitored by in vivo bioluminescent imaging. Mice were observed over 60 days and their survival rates recorded on a Kaplan-Meier survival curve.

Mice having xenografted GBM established in accordance with the procedures described above were randomly divided into 3 groups: control (vehicle), TMZ (5 or 20 mg/kg) group, and TMZ + VEGF group; and tumor size and survival time course were measured.

Figure 3 shows the tumor volume time course when GBM mice were treated with either (A) 5 mg/Kg TMZ or (B) 20 mg/Kg TMZ, in the absence or presence of low dose VEGF pre-treatment. 5 mg/kg TMZ was found to be sufficient to delay the growth of GBM for at least 20 days (as compared to GBM mice not treated by TMZ), and pre-treatment with VEGF (0.3 µg/kg) further enhanced the delay in tumor growth by TMZ. Similar results were observed when the mice were treated with 20 mg/kg TMZ (Figure 3, panel B). High dose TMZ (20 mg/Kg) did not exhibit improved treatment effect as compared with the low dose TMZ treatment (5 mg/kg) .

The survival rates of the tested animals are summarized in Table 1.

**Table 1. Low Dose VEGF Pre-treatment Improved GBM Mice Survival Rate**

| **Treatment** | **Replicates (n=)** | **Median Survival (days)** | **Median Survival vs vehicle (% increase)** | **Median Survival with VEGF vs TMZ alone (% increase)** |
|---|---|---|---|---|
| **Sham Operation** | 4 | >60 | n/a | |
| **Vehicle Control** | 3 | 38 | = C3/C3^{∗}100 \# | |
| | | | "0.00" \* | |
| | | | MERGEFORMAT | |
| | | | 100.00 | |
| **TMZ 5mg/kg** | 5 | 50 | 31.58 | |
| **TMZ 5mg/kg + VEGF** | 4 | 55 | 44.74 | 10.00 |
| **TMZ 20mg/kg** | 3 | 50 | 31.58 | |
| **TMZ 20mg/kg + VEGF** | 2 | 52 | 36.84 | 4.00 |

In sum, VEGF pre-treatment at a low dose improved the anti-GBM effect of TMZ, which manifests in the increase of life span of the tested animals. The median survival rate is 10% higher than that treated by 5mg/Kg TMZ alone.

Further, the effect of low dose VEGF pre-treatment on the delivery of anti-cancer drug doxorubicin to six vital organs, brain, lung, liver, kidney, spleen, and heart, was investigated.

Animals used were 12 week old, male, BALB/c NU mice, who had been previously 6 implanted with 3x10 luciferase-expressing U87 glioblastoma cells. The tumor was allowed to progress for three weeks and was confirmed by IVIS prior to VEGF/control/Doxorubicin administration. Doxorubicin (8mg/kg) was administered 3 hours post VEGF/control pre-treatment. Animals were perfused systemically with 50ml normal saline, organs were collected and doxorubicin was extracted from tissues and quantified by HPLC. A significant increase in Doxorubicin measured in the brain was detected following VEGF treatment, with no changes in other vital organs. n = 3 per group. Figure 6. These results indicate that low dose VEGF pre-treatment facilitated drug delivery across the BBB to the brain, but not to other organs.

### Example 4: VEGF Pre-Treatment Enhances Brain Imaging

Brain imaging is one of the key factors in diagnosing a CVA. Imaging is used to confirm the location and size of infarcted areas and also to rule out other insults which may present with similar symptoms such as brain tumour or inter-cerebral haemorrhage. In the US, the current first-line imaging method for suspected stroke is noncontrast computed tomography (CT), but computed tomography angiogram (CTA) utilising injected contrast agent is becoming more commonly used. Birenbaum et al., Western Journal of Emergency Medicine 12(1); 2011.

CTA is a more powerful technique, allowing 3D images of blood vessels to be captured over time, visualising arterial blockages more precisely. This information is extremely useful for medical teams when deciding whether to prescribe thrombolytic agents and essential for surgical teams when attempting mechanical clot removal angiography.

Contrast agents are used to improve visualisation of blood vessels. Lower doses are suitable for visualising large arteries such as the aorta, but higher doses are required for smaller arteries. However, these agents are expensive and also have some toxicity, being linked to kidney damage and being contraindicated in some patients.

Provided herein are experimental data demonstrating that VEGF pre-treatment increased the detectable levels of subsequently administered contrast agent in the brain. Such an increase allows for a reduced dose of contrast agent to be used for brain imaging, thus reducing cost, risk of side effects and potentially allowing better visualisation of small arterioles in the brain.

Briefly, 3 mice (FVB mice, 25g, 8 weeks old) were injected intravenously (tail vein) with 0.3ug/kg VEGF. 45 minutes later, the mice were injected with 0.2mmol/kg gadolinium contrast agent. Brain images were taken after VEGF injection but before contrast agent injection, and then T1 and T2 weighted images were taken 5 minutes after contrast agent injection. Mice injected with normal saline were used as controls (Figure 8).

Three replicates of the VEGF-treated mice, consistently demonstrated a statistically significant increase in the level of gadolinium contrast agent detected in the brain. The signal to noise ratio (SNR) was enhanced by 17%, 14% and 15.5% after VEGF treatment for each of the 3 mice, respectively. This was consistent for the cortex, striatum and for both sides of the brain. In a healthy control mice, any contrast enhancement did not exceed 5.0%. The results demonstrate an average increase in the detectable level of contrast agent of 15.5% following VEGF pre-treatment (Table 2).

**Table 2. Low Dose VEGF Pre-treatment Improved Detectable the Detectable Levels of Gadolinium Contrast Agent in the Brain**

| **Cortex, signal to noise ratio** | **T1WI Pre-iv** | **T1WI Post-iv** | **Increase %** |
|---|---|---|---|
| **VEGF** | **50.48** | 58.30 | 15.5 (P<0.05) |
| **Control** | **53.70** | 56.12 | **4.5 (n/s)** |

The 7T MRI scanning parameters (Pharmascan 7T 16-cm bore horizontal MRI system) used in the experiments are as follows:
SE-T1WI parameters: TR= 400 ms; TE =10.8 ms; FOV=2x2 cm; NEX =8; Slice-thickness = 0.8mm, 16slices; Time= 6min49sec; Matrix= 256^{∗}128 reco to 256^{∗}256.
FSE-T2WI parameters: TR= 4000 ms; TEeff =70 ms; FOV=2x2 cm; NEX =4; Slice-thickness = 0.8mm, 16slices; Time= 4min16sec; Matrix= 256^{∗}128 reco to 256^{∗}256.

It will be understood that the description of embodiments provided herein is given by way of example only and that various modifications may be made by those with ordinary skill in the art.

Provided below are a number of specific examples:
A method of facilitating the delivery of an agent across blood-brain barrier (BBB) of a subject comprising administering to the subject in sequence or concomitantly, (i) an effective amount of a growth factor selected from the group consisting of, vascular endothelial growth factor (VEGF), insulin-like growth factor I (IGF-1), IGF-II, a portion thereof and a combination thereof; and (ii) the agent, which is any of a therapeutic agent or an imaging agent; wherein the administered amount of the growth factor is capable of transiently increasing BBB permeability of the subject and thereby allowing the agent to be delivered across BBB.

A method of treating a subject suffering from a brain tumor, a brain stroke, a neuropsychiatric disorder and/or a neurodegenerative disease, comprising: administering to the subject in sequence or concomitantly, a first effective amount of a growth factor selected from the group consisting of, vascular endothelial growth factor (VEGF), insulin-like growth factor I (IGF-1), IGF-II, a portion thereof and a combination thereof; and a second effective amount of a therapeutic agent; so as to ameliorate one or more symptoms related to the brain tumor, the brain stroke, the neuropsychiatric disorder, and/or the neurodegenerative disease.

In any of the above methods, growth factor is administered in the amount of 5 to 200 ng/kg. The imaging agent is a contrast agent for computed tomography (CT) or magnetic resonance imaging (MRI). The therapeutic agent is an anti-cancer drug, an anti-psychotic, an anti-coagulant, a protein or a stem cell. The anti-cancer drug is an alkylating agent, a topoisomerase inhibitor, an anti-metabolite, or a cytotoxicity antibiotic.

The alkylating agent can be cisplatin, carboplatin, oxaliplatin, mechlorethamine, cyclophosphamide, melphalan, chlorambucil, ifosfamide, busulfan, N-nitroso-N-methylurea (MNU), carmustine, lomustine, semustine, fotemustine, streptozotocin, dacarbazine, mitozolomide, temozolomide, thiotepa, mytomycin, or diaziquone.

The topoisomerase inhibitor can be camptothecin, irinotecan, topotecan, etoposide, doxorubicin, teniposide, novobiocin, merbarone, or aclarubicin.

The anti-metabolite can be fluoropymidine, deoxynucleoside analogue, thiopurine, methotrexate, or pemetrexed.

The cytotoxicity antibiotic can be actinomycin, bleomycin, plicamycin, mitomycin, doxorubicin, daunorubicin, epirubicin, idarubicin, piraubicin, alcarubicin, or mitoxantrone.

The anti-coagulant can be aspirin, clopidoqrel, dipyridamole, warfarin or heparin.

The protein can be tissue plasminogen activator (TPA).

The anti-psychotic can be butyrophenone, phenothiazine, fluphenazine, perphenazine, prochlorperazine, thioridazine, trifluoperazine, mesoridazine, promazine, triflupromazine, levomepromazine, promethazine, thioxanthene, chlorprothixene, flupenthixol, thiothixene, zuclopenthixol, clozapine, olanzapine, risperidone, quetiapine, ziprasidone, amisulpride, asenapine, paliperidone, aripiprazole, a lamotrigine, memantine, tetrabenazine, cannabidiol, LY2140023, Droperidol, Pimozide, Butaperazine, Carphenazine, Remoxipride, Piperacetazine, Sulpiride, acamprosate, tetrabenazine, benzoic acid, sodium benzoate, potassium benzoate, calcium benzoate, lithium benzoate, 2-aminobenzoate, 3 aminobenzoate, or 4-aminobenzoate.

The stem cell is a meschymal stem cell (MSC).

The anti-dementia agent is memantine or an acetylcholinesterase inhibitor (AChEI), which can be galantamine, tacrine, donepezil, rivastigmine, huperzine A, zanapezil, ganstigmine, phenserine, phenethylnorcymserine, cymserine, thiacymserine, SPH 1371, ER 127528, RS 1259 or a mixture thereof.

The neurodegenerative disease can be Alzhemer's disease (AD), argyrophilic grain disease, amyotrophic lateral sclerosis (ALS), ALS-parkinsonism dementia complex of Guam, vascular dementia, frontotemporal dementia, semantic dementia, dementia with Lewy bodies, Huntington's disease, inclusion body myopathy, inclusion body myositis, or Parkinson's disease (PD).

The neuropsychiatric disorder can be schizophrenia, delirium, Alzheimer's disease (AD), depression, mania, attention deficit disorders (ADD), attention deficit hyperactivity disorder (ADHD), drug addiction, mild cognitive impairment, dementia, agitation, apathy, anxiety, psychoses, post-traumatic stress disorders, irritability, or bipolar disorder.

A method of imaging a brain area of a subject comprising: (i) administering to the subject in sequence or concomitantly, an effective amount of a growth factor selected from the group consisting of, vascular endothelial growth factor (VEGF), insulin-like growth factor I (IGF-1), IGF-II, a portion thereof and a combination thereof; and a sufficient amount of a contrast agent for computed tomography (CT) or magnetic resonance imaging (MRI); and (ii) monitoring the distribution of the contrast agent as it moves through the brain area. The growth factor is administered in the amount of 5 to 200 ng/Kg.

## Claims

1. Vascular endothelial growth factor (VEGF) for use in treating a brain tumor in a subject, wherein an effective amount of VEGF is to be administered systemically to the subject, and wherein an anti-cancer agent is to be administered systemically to the subject within 5 hours after the administration of VEGF.

2. VEGF for use according to claim 1, wherein the effective amount of VEGF is 5 to 200 ng/kg, preferably 25 ng/kg.

3. VEGF for use according to any one of claims 1 or 2, wherein the anti-cancer agent is to be administered 15 to 180 minutes, preferably 45 minutes or 3 hours, after the administration of VEGF.

4. VEGF for use according to any one of claims 1-3, wherein the anti-cancer agent is an alkylating agent, a topoisomerase inhibitor, an anti-metabolite, a cytotoxicity antibiotic, or a biologic.

5. VEGF for use according to claim4, wherein the alkylating agent is selected from the group consisting of cisplatin, carboplatin, oxaliplatin, mechlorethamine, cyclophosphamide, melphalan, chlorambucil, ifosfamide, busulfan, N-nitroso-N-methylurea (MNU), carmustine, lomustine, semustine, fotemustine, streptozotocin, dacarbazine, mitozolomide, temozolomide, thiotepa, mytomycin, and diaziquone.

6. VEGF for use according to claim 4, wherein the topoisomerase inhibitor is selected from the group consisting of, camptothecin, irinotecan, topotecan, etoposide, doxorubicin, teniposide, novobiocin, merbarone, and aclarubicin.

7. VEGF for use according to claim 4, wherein the anti-metabolite is selected from the group consisting of, fluoropymidine, deoxynucleoside analogue, thiopurine, methotrexate, and pemetrexed.

8. VEGF for use according to claim 4, wherein the cytotoxicity antibiotic is selected from the group consisting of, actinomycin, bleomycin, plicamycin, mitomycin, doxorubicin, daunorubicin, epirubicin, idarubicin, piraubicin, alcarubicin, and mitoxantrone.

9. VEGF for use according to claim 4, wherein the biologic is selected from the group consisting of, Bevacizumab, Cetuximab, Pemtumomab, oregovomab, minretumomab, Etaracizumab, Volociximab, Cetuximab, panitumumab, nimotuzumab, Trastuzumab, pertuzumab, AVE1642, IMC-A12, MK-0646, R1507, CP 751871, Mapatumumab, KB004 and IIIA4.

10. A kit comprising:
(i) a first container containing a first formulation that comprises a vascular endothelial growth factor (VEGF), and
(ii) a second container containing a second formulation that comprises an anti-cancer agent.

11. Vascular endothelial growth factor (VEGF) for use in facilitating delivery of an agent across the blood-brain barrier of a subject, wherein an effective amount of VEGF is to be administered systemically to a subject within 5 hours prior to administration of the agent, wherein the effective amount of VEGF is 5 to 200 ng/kg, and wherein the agent is a therapeutic agent or a diagnostic agent.

12. VEGF for use according to claim 11, wherein the effective amount of VEGF is 25 ng/kg.

13. VEGF for use according to claim 11 or claim 12, wherein the VEGF is to be administered 15 minutes to 3 hours, preferably 45 minutes or 3 hours, prior to the administration of the agent.

14. VEGF for use according to any one of claims 11-13, wherein the subject is a human patient having, suspected of having, or at risk for a brain disease, preferably wherein the brain disease is selected from the group consisting of ischemic stroke, a neurodegenerative disease, and a neuropsychiatric disorder.

15. VEGF for use according to any one of claims 11-14, wherein the agent is a diagnostic contrast agent.

## Patentansprüche

1. Vaskulärer endothelialer Wachstumsfaktor (VEGF) zur Verwendung in der Behandlung eines Hirntumors in einer Person, wobei eine wirksame Menge an VEGF an die Person systemisch verabreicht werden soll und wobei ein Antikrebsmittel an die Person innerhalb von 5 Stunden nach der Verabreichung von VEGF systemisch verabreicht werden soll.

2. VEGF zur Verwendung gemäß Anspruch 1, wobei die wirksame Menge an VEGF 5 bis 200 ng/kg, vorzugsweise 25 ng/kg beträgt.

3. VEGF zur Verwendung gemäß einem der Ansprüche 1 oder 2, wobei das Antikrebsmittel 15 bis 180 Minuten, vorzugsweise 45 Minuten oder 3 Stunden nach der Verabreichung von VEGF verabreicht werden soll.

4. VEGF zur Verwendung gemäß einem der Ansprüche 1 bis 3, wobei das Antikrebsmittel ein Alkylierungsmittel, ein Topoisomerase-Hemmer, ein Antimetabolit, ein zytotoxisches Antibiotikum oder ein Biologikum ist.

5. VEGF zur Verwendung gemäß Anspruch 4, wobei das Alkylierungsmittel aus der Gruppe ausgewählt ist, bestehend aus Cisplatin, Carboplatin, Oxaliplatin, Mechlorethamin, Cyclophosphamid, Melphalan, Chlorambucil, Ifosfamid, Busulfan, N-Nitroso-N-methylurea (MNU), Carmustin, Lomustin, Semustin, Fotemustin, Streptozotocin, Dacarbazin, Mitozolomid, Temozolomid, Thiotepa, Mytomycin und Diaziquon.

6. VEGF zur Verwendung gemäß Anspruch 4, wobei der Topoisomerase-Hemmer aus der Gruppe ausgewählt ist, bestehend aus Camptothecin, Irinotecan, Topotecan, Etoposid, Doxorubicin, Teniposid, Novobiocin, Merbaron und Aclarubicin.

7. VEGF zur Verwendung gemäß Anspruch 4, wobei der Antimetabolit aus der Gruppe ausgewählt ist, bestehend aus Fluoropymidin, Desoxynukleosid-Analogon, Thiopurin, Methotrexat und Pemetrexed.

8. VEGF zur Verwendung gemäß Anspruch 4, wobei das zytotoxische Antibiotikum aus der Gruppe ausgewählt ist, bestehend aus Actinomycin, Bleomycin, Plicamycin, Mitomycin, Doxorubicin, Daunorubicin, Epirubicin, Idarubicin, Piraubicin, Alcarubicin und Mitoxantron.

9. VEGF zur Verwendung gemäß Anspruch 4, wobei das Biologikum aus der Gruppe ausgewählt ist, bestehend aus Bevacizumab, Cetuximab, Pemtumomab, Oregovomab, Minretumomab, Etaracizumab, Volociximab, Cetuximab, Panitumumab, Nimotuzumab, Trastuzumab, Pertuzumab, AVE1642, IMC-A12, MK-0646, R1507, CP 751871, Mapatumumab, KB004 und IIIA4.

10. Ein Kit umfassend:
(i) einen ersten Behälter, enthaltend eine erste Formulierung, die einen vaskulären endothelialen Wachstumsfaktor (VEGF) umfasst, und
(ii) einen zweiten Behälter, enthaltend eine zweite Formulierung, die ein Antikrebsmittel umfasst.

11. Vaskulärer endothelialer Wachstumsfaktor (VEGF) zur Verwendung zur Erleichterung des Transports eines Mittels durch die Blut-Hirn-Schranke einer Person, wobei eine wirksame Menge an VEGF innerhalb von 5 Stunden vor Verabreichung des Mittels an eine Person systemisch verabreicht werden soll, wobei die wirksame Menge an VEGF 5 bis 200 ng/kg beträgt und wobei das Mittel ein Therapeutikum oder Diagnostikum ist.

12. VEGF zur Verwendung gemäß Anspruch 11, wobei die wirksame Menge an VEGF 25 ng/kg beträgt.

13. VEGF zur Verwendung gemäß Anspruch 11 oder Anspruch 12, wobei der VEGF 15 Minuten bis 3 Stunden, vorzugsweise 45 Minuten oder 3 Stunden vor der Verabreichung des Mittels verabreicht werden soll.

14. VEGF zur Verwendung gemäß einem der Ansprüche 11 bis 13, wobei die Person ein Humanpatient ist, der eine Hirnerkrankung aufweist, vermutlich aufweist oder ein Risiko für eine Hirnerkrankung aufweist, wobei vorzugsweise die Hirnerkrankung aus der Gruppe ausgewählt ist, bestehend aus ischämischem Schlaganfall, einer neurodegenerativen Erkrankung und einer neuropsychiatrischen Störung.

15. VEGF zur Verwendung gemäß einem der Ansprüche 11 bis 14, wobei das Mittel ein diagnostisches Kontrastmittel ist.

## Revendications

1. Facteur de croissance de l'endothélium vasculaire (VEGF) pour une utilisation dans le traitement d'une tumeur cérébrale chez un sujet, dans lequel une quantité efficace de VEGF est destinée à être administrée par voie systémique au sujet, et dans lequel un agent anticancéreux est destiné à être administré par voie systémique au sujet dans les 5 heures suivant l'administration du VEGF.

2. VEGF pour une utilisation selon la revendication 1, dans lequel la quantité efficace de VEGF est de 5 à 200 ng/kg, de préférence de 25 ng/kg.

3. VEGF pour une utilisation selon l'une quelconque des revendications 1 et 2, dans lequel l'agent anticancéreux est destiné à être administré 15 à 180 minutes, de préférence 45 minutes ou 3 heures, après l'administration du VEGF.

4. VEGF pour une utilisation selon l'une quelconque des revendications 1 à 3, dans lequel l'agent anticancéreux est un agent d'alkylation, un inhibiteur de topoisomérase, un antimétabolite, un antibiotique cytotoxique, ou un agent biologique.

5. VEGF pour une utilisation selon la revendication 4, dans lequel l'agent d'alkylation est choisi dans le groupe constitué par le cisplatine, le carboplatine, l'oxaliplatine, la méchloréthamine, le cyclophosphamide, le melphalan, le chlorambucil, l'ifosfamide, le busulfan, la N-nitroso-N-méthylurée (MNU), la carmustine, la lomustine, la sémustine, la fotémustine, la streptozotocine, la dacarbazine, le mitozolomide, le témozolomide, le thiotépa, la mytomycine, et la diaziquone.

6. VEGF pour une utilisation selon la revendication 4, dans lequel l'inhibiteur de topoisomérase est choisi dans le groupe constitué par la camptothécine, l'irinotécan, le topotécan, l'étoposide, la doxorubicine, le téniposide, la novobiocine, la merbarone, et l'aclarubicine.

7. VEGF pour une utilisation selon la revendication 4, dans lequel l'antimétabolite est choisi dans le groupe constitué par la fluoropymidine, un analogue de désoxynucléoside, la thiopurine, le méthotrexate, et le pémétrexed.

8. VEGF pour une utilisation selon la revendication 4, dans lequel l'antibiotique cytotoxique est choisi dans le groupe constitué par l'actinomycine, la bléomycine, la plicamycine, la mitomycine, la doxorubicine, la daunorubicine, l'épirubicine, l'idarubicine, la piraubicine, l'alcarubicine, et la mitoxantrone.

9. VEGF pour une utilisation selon la revendication 4, dans lequel l'agent biologique est choisi dans le groupe constitué par le bévacizumab, le cétuximab, le pemtumomab, l'orégovomab, le minrétumomab, l'étaracizumab, le volociximab, le cétuximab, le panitumumab, le nimotuzumab, le trastuzumab, le pertuzumab, AVE1642, IMC-A12, MK-0646, R1507, CP751871, le mapatumumab, KB004 et IIIA4.

10. Trousse comprenant :
(i) un premier récipient contenant une première formulation qui comprend un facteur de croissance de l'endothélium vasculaire (VEGF), et
(ii) un deuxième récipient contenant une deuxième formulation qui comprend un agent anticancéreux.

11. Facteur de croissance de l'endothélium vasculaire (VEGF) pour une utilisation afin de faciliter la délivrance d'un agent à travers la barrière hémato-encéphalique d'un sujet, dans lequel une quantité efficace de VEGF est destinée à être administrée par voie systémique à un sujet dans les 5 heures précédant l'administration de l'agent, dans lequel la quantité efficace de VEGF est de 5 à 200 ng/kg, et dans lequel l'agent est un agent thérapeutique ou un agent diagnostique.

12. VEGF pour une utilisation selon la revendication 11, dans lequel la quantité efficace de VEGF est de 25 ng/kg.

13. VEGF pour une utilisation selon la revendication 11 ou la revendication 12, lequel VEGF est destiné à être administré 15 minutes à 3 heures, de préférence 45 minutes ou 3 heures, avant l'administration de l'agent.

14. VEGF pour une utilisation selon l'une quelconque des revendications 11 à 13, dans lequel le sujet est un patient humain ayant, suspecté d'avoir, ou risquant d'avoir, une maladie cérébrale, de préférence dans lequel la maladie cérébrale est choisie dans le groupe constitué par un accident vasculaire ischémique, une maladie neurodégénérative, et un trouble neuropsychiatrique.

15. VEGF pour une utilisation selon l'une quelconque des revendications 11 à 14, dans lequel l'agent est un agent de contraste diagnostique.
